# EUROPÄISCHE PATENTSCHRIFT

(11) **EP 2 374 494 B1**
(45) Veröffentlichungstag und Bekanntmachung des Hinweises auf die Patenterteilung: **01.03.2017**
(21) Anmeldenummer: 10186319.9
(22) Anmeldetag: 30.12.2005
(51) Int. Cl.: A61M 16/06

(54) **Nasenstück für Luftbrille**
Nose piece for nasal cannula
Embout nasal pour lunette à air

(30) Priorität: 07.01.2005 DE 102005000922
(43) Veröffentlichungstag der Anmeldung: 12.10.2011
(62) Teilanmeldung aus: 07113959.6
(73) Patentinhaber: TNI medical AG, 97084 Würzburg (DE)
(72) Erfinder: Baecke, Martin, 06847 Dessau (DE); Schöbel, Ulla, 06366 Köthen (DE); Kilz, Silvio, 06785 Oranienbaum-Wörlitz (DE); Krause, Heiko, 06779 Schierau (DE); Müller, Ingo, Dr., 01097 Dresden (DE)
(74) Vertreter: advotec.

(56) Entgegenhaltungen:
- WO-A1-00/64521
- WO-A1-00/72905
- WO-A1-01/89381
- WO-A1-03/068301
- WO-A1-2004/105848
- WO-A2-02/062413
- DE-A1- 19 942 748
- FR-A1- 2 827 778
- US-A- 4 989 599
- US-A1- 2003 079 749
- US-A1- 2004 050 390
- US-A1- 2004 182 392

## Beschreibung

Die Erfindung bezieht sich auf das technische Gebiet von Luftbrillen gemäß dem Oberbegriff des Patentanspruchs 1, von Nasenstücken hierfür gemäß den Oberbegriffen der Patentansprüche 12 bis 15, von Y-Stücken gemäß dem Oberbegriff des Patentanspruchs 16 sowie von Verfahren gemäß dem Oberbegriff von Patentanspruch 19. Insbesondere bezieht sich die Erfindung auf konstruktive Änderungen, die den Einsatz von Luftbrillen bei der pneumatischen Schienung der oberen Atemwege erleichtern.

Obstruktive Atmungsstörungen führen zu Apnoen (Atemstillstand), durch die der Schlafende erwacht. Häufige Apnoen verhindern, dass der Schlafende in den erholsamen Tiefschlaf fällt. Menschen, die Apnoen während des Schlafens erleiden, sind deshalb tagsüber unausgeschlafen, was zu sozialen Problemen am Arbeitsplatz und im schlimmsten Fall zu tödlichen Unfällen, beispielsweise bei Berufskraftfahrern, führen kann.

Im Stand der Technik sind Geräte zur Durchführung der CPAP (continuous positive airway pressure)-Therapie bekannt. Die CPAP-Therapie wird in Chest. Vol. 110, Seiten 1077 bis 1088, Oktober 1996 und Sleep, Vol. No. 19, Seiten 184 bis 188 näher beschrieben.

In der CPAP-Therapie wird einem Patienten ein konstanter positiver Druck über eine Nasenmaske zugeführt, um die oberen Atemwege zu schienen. Bei richtiger Wahl des Überdrucks gewährleistet dieser, dass die oberen Atemwege während der gesamten Nacht vollständig geöffnet bleiben und somit keine obstruktiven Atemstörungen auftreten. Unter anderem zur Komfortsteigerung wurden BiLevel-Geräte entwickelt, die den Druck während der aus Atempause absenken. Als Oberbegriff für Geräte zur pneumatischen Schienung der oberen Atemwege wird hier der Begriff PAP-Geräte verwendet.

Schnarchen und Apnoen können die gleiche Ursache haben, nämlich zu schlaffes Gaumen- und Zungengewebe.

Aus dem Stand der Technik sind ferner Sauerstoffbrillen für die Sauerstoffbehandlung bekannt. Mit der Sauerstoffbrille wird dem Patienten Luft mit einem erhöhten Sauerstoffpartialdruck (> 210 mbar) oder reiner Sauerstoff in die Nase appliziert. Eine Sauerstoffbehandlung findet zum Beispiel bei akuter oder chronischer Hypoxämie infolge Atem- oder Herz-Kreislaufstörung (Myokardinfarkt, Schock) oder bestimmten Vergiftungen, zum Beispiel durch Kohlenmonoxid, Kohlendioxid, Leuchtgas oder Rauch statt.

Aus der WO 02/062413 A2 (HEWO1) ist der Einsatz von Sauerstoffbrillen in einem Antischnarchgerät bekannt. Sauerstoffbrillen werden in diesem Zusammenhang als Luftbrillen bezeichnet.

Vapotherm 2000i ist ein Befeuchtungssystem, das Luftflüsse im Bereich von 8 bis 40 l/min über Luftbrillen (nasal cannula) an Patienten liefert. Die geförderte Luft wird befeuchtet und beheizt. Luft kann mit Sauerstoff angereichert werden.

Die WO0072905A1 beschreibt eine Luftbrille mit einem Nasenstück, welches ein die beiden Zinken zur Luftapplikation mechanisch und pneumatisch verbindendes zentrales Verbindungsstück aufweist.

Es ist Aufgabe der Erfindung, eine Luftbrille, ein Nasenstück, ein Y-Stück sowie ein Verfahren anzugeben, die besonders gut zur pneumatischen Schienung der oberen Atemwege geeignet sind.

Diese Aufgabe wird durch die Lehre der unabhängigen Ansprüche gelöst.

Bevorzugte Ausführungsformen der Erfindung sind Gegenstand der Unteransprüche.

Das Beheizen eines Gabelschlauches mittels eines Heizdrahtes kann die Kompensation von Feuchtigkeit im Gabelschlauch verhindern. Eine Verlegung des Heizdrahtes im Inneren des Gabelschlauches ist fertigungstechnisch einfach. Aufgrund der Wärmeabgabe an die Umgebung des Gabelschlauches sinkt die Temperatur im Gabelschlauch näherungsweise linear mit dem Abstand vom Kompressor. Dieser Temperaturabfall kann durch eine konstante Heizleistung pro Längeneinheit, wie sie eben ein Heizdraht erzeugt, ausgeglichen werden. Bedingt durch die Konstruktion des Schlauches lässt sich die erforderliche Heizleistung für die gesamte Luftbrille kleiner als 15 Watt halten. Andernfalls wären aufgrund von gesetzlichen Vorgaben der Einsatz von brandhemmenden Kunststoffen erforderlich, die in der Regel nicht biokompatibel sind und deren Einsatz deshalb in medizintechnischen Produkten problematisch ist.

Eine Messung der Temperatur der applizierten Luft erlaubt es, die Heizleistung eines Heizdrahtes oder einer Heizung in einem Kompressorgehäuse so zu steuern, dass die Temperatur vom Benutzer als angenehm empfunden wird. Ohne Kompensation des Temperaturabfalls im Gabelschlauch wären die Applikationsöffnungen in den Zinken die kältesten Orte. Folglich wird hier am ehesten Luftfeuchtigkeit kondensieren. Aus diesem Grund ist eine Heizleistungssteuerung aufgrund einer Temperaturmessung in der Nähe der Applikationsöffnungen am besten geeignet, Kondensation in der gesamten Luftbrille zu verhindern.

Aus Gründen der Materialeinsparung ist es wünschenswert, den Temperatursensor auch über den Heizdraht auszulesen. Aufgrund der Fortschritte der Integration von Schaltkreisen ist es möglich, digitale Temperatursensoren mit einer akzeptablen Baugröße herzustellen, die ihr Sensorsignal auf den Heizdraht aufmodulieren.

Das Abweichen der äußeren Mantelfläche der Isolierung des Heizdrahts von der üblichen Zylinderform durch Erhöhungen und Vertiefungen verhindert, dass bei einem Abknicken des Gabelschlauches der Luftstrom durch den Gabelschlauch zu stark reduziert wird. In so einem Fall besteht die Gefahr, dass der Heizdraht an der Knickstelle überhitzt und in den Gabelschlauch einschmilzt, weil der Heizdraht an der Knickstelle unzureichend gekühlt wird.

Besonders geeignet zur Sicherstellung eines ausreichenden Luftflusses, falls der Gabelschlauch abknickt, sind entlang des Heizdrahtes verlaufende Erhöhungen und Vertiefungen. Ein dreieckförmiger Querschnitt der Erhöhungen sorgt in vorteilhafter Weise dafür, dass die Berührungsfläche zwischen Isolierung des Heizdrahtes und Innenseite des Gabelschlauches sowohl im normalen Betrieb als auch beim Abknicken klein bleibt. Der insgesamt sternförmige Querschnitt der Isolierung vergrößert vorteilhafterweise die Oberfläche der Isolierung und sorgt damit für eine Verringerung des thermischen Widerstands zwischen der Isolierung und der vorbeiströmenden Luft.

Auch in Längsrichtung des Gabelschlauches verlaufende Vorsprünge sorgen in vorteilhafter Weise dafür, dass selbst dann ein ausreichender Luftstrom unter anderem zur Kühlung des Heizdrahtes sichergestellt ist, falls der Gabelschlauch abknickt.

Stabilisierungsfäden dienen dazu, eine Längsdehnung der Schläuche zu reduzieren.

Die mechanische Verbindung zweier Stücke des Gabelschlauches an ihrem steckerseitigen Ende erlaubt es, ein Y-Stück einzusparen oder dies in den Stecker zu integrieren. Dies führt in vorteilhafter Weise zu einer Reduzierung der Schallemission, weil das in den Stecker integrierte Y-Stück weiter von den Applikationsöffnungen entfernt ist.

Innenradiusstufen an unterschiedlichen Verbindungsstellen können gerade die Schlauchstärke kompensieren, sodass nach Befestigung eines Schlauches der Übergang zwischen Schlauch und dem entsprechenden Bauteil glatt ist. An einem glatten Übergang entstehen weniger Wirbel und damit weniger Schall.

Auch die Übergangsbereiche zwischen einem Zinken und dem zentralen Verbindungsstück sowie einem Zinken und dem Verbindungsstück auf der Seite des Zinkens sind abgerundet, um einer Wirbelbildung und damit einer Geräuschemission in vorteilhafter Weise vorzubeugen.

Durch eine Einbuchtung im zentralen Verbindungsstück kann ein optimaler Strömungswiderstand des Verbindungsstücks eingestellt werden.

Im Folgenden wird eine bevorzugte Ausführungsform der Erfindung unter Bezugnahme auf die beiliegenden Zeichnungen näher erläutert. Dabei zeigen:
- Fig. 1: eine erfindungsgemäße Luftbrille mit einer ersten Ausführungsform eines Nasenstücks;
- Fig. 2: ein Y-Stück mit einem Temperatursensor;
- Fig. 3: eine erfindungsgemäße Luftbrille mit einem Doppellumenschlauch;
- Fig. 4: eine Schaltung zur Temperaturmessung;
- Fig. 5: den Querschnitt eines Heizdrahtes;
- Fig. 6: den Querschnitt eines Schlauches mit Heizdraht;
- Fig. 7: eine perspektivische Ansicht einer zweiten Ausführungsform eines Nasenstücks aus einer ersten Richtung;
- Fig. 8: einen Schnitt durch eine Zinke entlang Z-Z;
- Fig. 9: eine perspektivische Ansicht der zweiten Ausführungsform des Nasenstücks aus einer zweiten Richtung;
- Fig. 10: einen Schnitt entlang M-M;
- Fig. 11: eine perspektivische Ansicht der zweiten Ausführungsform des Nasenstücks aus einer dritten Richtung; und
- Fig. 12: ein Y-Stück für eine erfindungsgemäße Luftbrille.

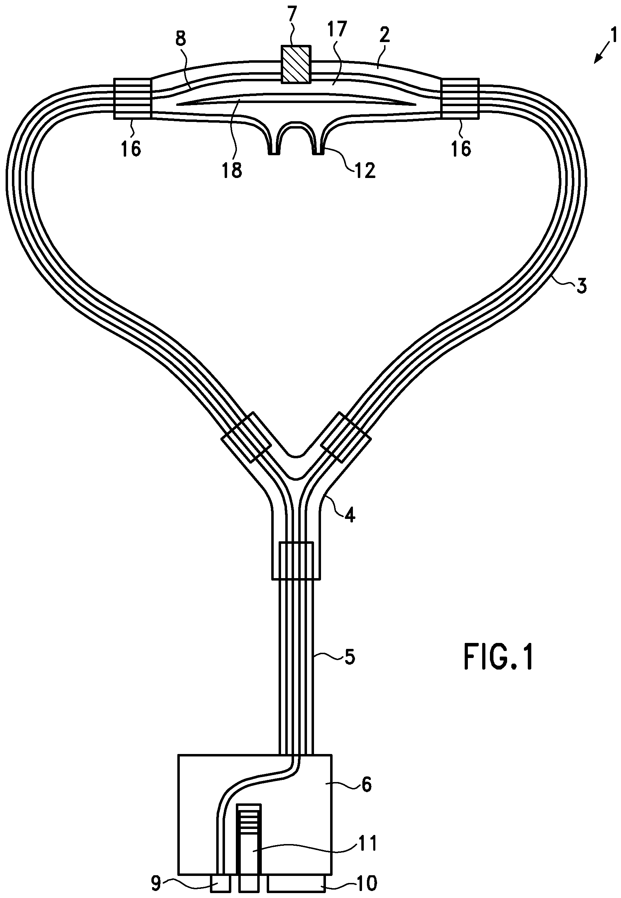

Fig. 1 zeigt eine erfindungsgemäße Luftbrille 1 mit einer ersten Ausführungsform eines Nasenstücks 2. Nasenstück 2 wird über einen Gabelschlauch 3, ein Y-Stück 4, einen Zuleitungsschlauch 5, sowie einen Stecker 6 mit komprimierter Luft versorgt. Nasenstück 2 weist zwei Zinken 12 zur Applikation von Luft in die beiden Nasenlöcher eines Benutzers auf. Innenradiusstufen 16 kompensieren den Unterschied zwischen Innen- und Außenradius der Gabelschläuche und verhindern damit sprunghafte Änderungen des Querschnitts der Luftwege.

Stecker 6 weist ein pneumatisches Steckerteil 10, ein elektrisches Steckerteil 9 auf sowie eine Klammer 11 auf. Vom elektrischen Steckerteil 9 führt ein Heizdraht 8 durch Zuleitungsschlauch 5, Y-Stück 4, das rechte Stück von Gabelschlauch 3, den rechten Teil vom Nasenstück 2 zu einem Temperatursensor 7 und von dort durch den linken Teil vom Nasenstück 2, das linke Stück von Gabelschlauch 3, Y-Stück 4 und Zuleitungsschlauch 5 zum elektrischen Steckerteil 9 zurück.

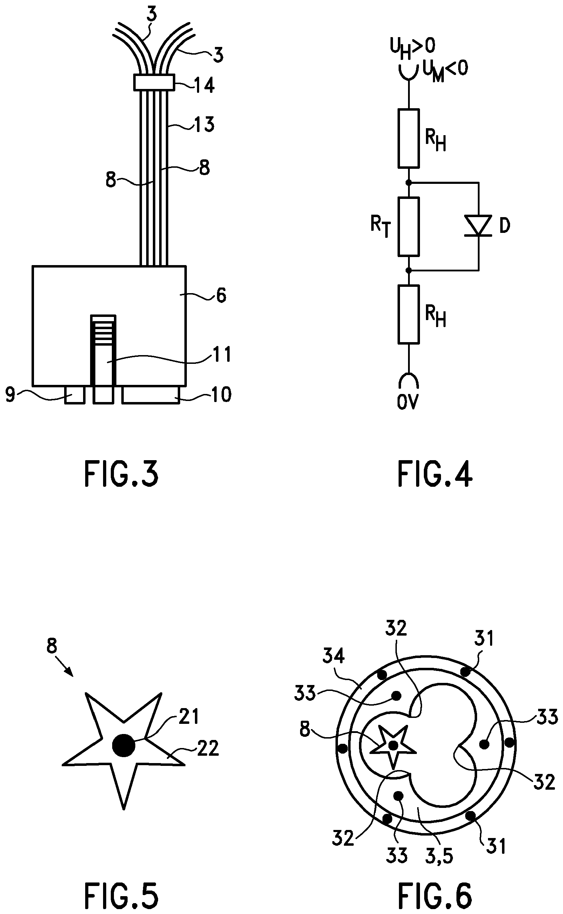

Die Klammer 11 rastet an einer für Stecker 6 vorgesehenen Buchse ein und sichert Stecker 6 gegen unabsichtliches Herausziehen. Ein möglicher Querschnitt von Gabelschlauch 3 und Zuleitungsschlauch 5 wird im Zusammenhang mit Fig. 6 erläutert. Zuleitungsschlauch 5 weist einen größeren Querschnitt als Gabelschlauch 3 auf, weil Zuleitungsschlauch 5 typischerweise den doppelten Luftfluss transportieren muss, die zu überbrückende Entfernung größer ist und die Komforteinbußen bei großer Schlauchdicke geringer sind. Das Wort Gabelschlauch wurde lediglich deshalb gewählt, weil sich Zuleitungsschlauch 5 bei Y-Stück 4 "aufgabelt".

Aus zulassungsrechtlicher Sicht kann es erforderlich werden, im Bereich des Nasenstücks 2 die Isolation von Heizdraht 8 durch eine zusätzliche Trennwand 18 von Zinken 12 abzuschirmen. Heizdraht 8 verläuft dann im Bereich von Nasenstück 2 in einem zusätzlichen Lumen 17.

Sollen Luftbrillen zur pneumatischen Schienung der oberen Atemwege benutzt werden, so besteht ein Problem in der Geräuschentwicklung aufgrund der hohen Luftflüsse durch die im Vergleich zu Beatmungsschläuchen dünnen Zuleitungsschläuche und Gabelschläuche. Hieraus resultiert eine hohe Strömungsgeschwindigkeit der Luft, die an Kanten zur Entstehung von Geräuschen führt. Deshalb wurde bei der in Figur 1 dargestellten Luftbrille darauf geachtet, dass die Innenwände des Zuleitungsschlauchs 5, des Y-Stücks 4, der beiden Stücke des Gabelschlauches 3, des Nasenstücks 2 sowie der Zinken 12 keine scharfen Kanten aufweisen und insbesondere die Innenseite der Übergänge zwischen diesen Bauteilen keine Stufen oder Kanten bilden.

Das Bauteil 7 kann in einer weiteren Ausführungsform ein Temperaturschalter 19 sein, den man als Temperatursensor mit schlechter Auflösung von einem Bit verstehen kann. Der Temperaturschalter kann beispielsweise durch einen Bimetallkontakt beispielsweise mit einer Auslösetemperatur im Bereich von 30°C bis 50°C, insbesondere von 40°C, realisiert werden. Falls die Temperatur des Temperaturschalters die Auslösetemperatur übersteigt, wird der Heizkreis unterbrochen.

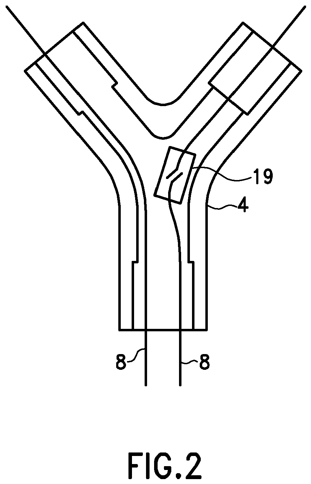

Zusätzlich oder alternativ zu Bauteil 7 kann ein Temperatursensor oder -schalter 19 im Y-Stück 4 untergebracht sein, wie dies in Fig. 2 dargestellt ist. Ein zusätzlicher Temperaturschalter, beispielsweise ein Bimetallkontakt mit einer Auslösetemperatur von (50±10)°C, kann eine weitere Sicherung gegen Überhitzen beispielsweise beim versehentlichen Abknicken des Gabelschlauchs 3 und/oder des Zuleitungsschlauchs 5 darstellen. Oberhalb der Auslösetemperatur wird der Heizkreis unterbrochen. Der in Fig. 2 dargestellte Temperaturschalter 19 stellt einen schematisierten Bimetallkontakt dar.

Falls im Nasenstück kein Temperatursensor 7 vorgesehen ist, kann ein Temperatursensor oder -schalter 19 allein Kondensation effektiv verhindern, da beim Y-Stück der nicht vom Körper des Patienten erwärmte Zuleitungsschlauch 5 endet. Insofern befindet sich im Zuleitungsschlauch 5 der kälteste und damit für Kondensation anfälligste Punkt zwischen Kompressor und Nasenstück 2. Wird die Temperatur des kältesten Punkts über dem Tau-Punkt gehalten, findet keine Kondensation statt. Eine Verlagerung des Temperatursensors oder Schalters in das Y-Stück 4 kann den Tragekomfort der Luftbrille 1 erhöhen, weil das Nasenstück 2 leichter und kleiner ausgeführt werden kann.

Da bei vorgegebener Geometrie der Luftbrille, insbesondere vorgegebenen Schlauchlängen und Durchmessern, aus der Temperatur im Y-Stück, der Heizleistung und dem eingestelltem Fluss näherungsweise die Lufttemperatur in den Zinken 12 berechnet werden kann, führt eine Verlagerung des Temperatursensors vom Nasenstück 2 in das Y-Stück 4 zu keinen nennenswerten Komforteinbussen.

Fig. 3 zeigt eine zweite Ausführungsform einer Luftbrille, bei der Zuleitungsschlauch 5 und Y-Stück 4 durch einen Doppellumenschlauch 13 ersetzt sind. Der Doppellumenschlauch besteht aus zwei Gabelschlauchstücken, die mechanisch miteinander verbunden sind. Bei dieser Ausführungsform entfällt das Y-Stück 4 oder ist gemäß einer anderen Sichtweise in den Stecker 6 integriert. Dort, wo die beiden Gabelschlauchstücke auseinander laufen, gibt es keine scharfen Kanten, sondern nur weite Radien. An dieser Stelle kann eine Schelle 14 vorgesehen sein, die ein weiteres aufspleißen des Doppellumenschlauchs verhindert. Die Aufteilung eines Luftstroms auf zwei Gabelschlauchstücke kann im Stecker 6 erfolgen und ist damit weiter von den Zinken 12 entfernt, sodass die Geräuschemission geringer ausfällt.

Fig. 4 zeigt eine Möglichkeit, einen Temperatursensor über nur zwei Heizdrähte auszulesen. Die beiden Heizdrähte 8 werden in dem in Figur 4 gezeigten Ersatzschaltbild durch die beiden Widerstände R_{H} dargestellt. R_{T} stellt einen Zweipol mit temperaturabhängigem Klemmenverhalten dar.

Im einfachsten Fall ist der Widerstand R_{T} lediglich ein temperaturabhängiger Widerstand wie beispielsweise ein Pt100 oder Pt1000. R_{T} ist groß gegenüber R_{H}. Die Heizdrähte haben typischerweise einen Widerstand von 15Ω mit großen Toleranzen. Wird an den drei in Serie geschalteten Widerständen eine positive Heizspannung U_{H} angelegt, so wird der Temperatursensor durch die parallel geschaltete Diode D kurz geschlossen, so dass im Wesentlichen nur die Heizdrähte beheizt werden. Wird an den drei in Serie geschalteten Widerständen eine negative oder eine kleine Messspannung U_{M} angelegt, so fällt der überwiegende Teil der Messspannung am Temperatursensor R_{T} ab. Hieraus kann die Temperatur des Temperatursensors bestimmt werden. Die verbleibenden Spannungsabfälle an den Heizwiderständen können herausgerechnet werden.

Es ist aber auch möglich, eine temperaturabhängige Stromquelle, wie sie zum Beispiel in Form der integrierten Schaltung AD592 vorliegt, als Zweipol R_{T} einzusetzen. In diesem Fall dient die Diode D dazu, die integrierte Schaltung für den Heizstrom zu überbrücken und damit zu schützen. Beispielsweise kann für die Diode D eine Schottky-Diode wegen ihrer geringen Durchlassspannung eingesetzt werden. Die Richtung des Messstroms ist dem Heizstrom entgegen gerichtet. Sein Betrag ist abhängig von der Temperatur und der eingesetzten integrierten Schaltung und beträgt wenige 100µA. Der besondere Vorteil dieser Lösung besteht darin, dass der Drahtwiderstand das Messergebnis praktisch nicht beeinflusst.

Neben den direkt analog übertragenden Sensoren ist auch die Umsetzung des Temperatursignals durch Aufmodulation auf den Heizstrom möglich. Dies kann sowohl analog als auch digital erfolgen und in kundenspezifischen Schaltkreisen realisiert werden. Solche Schaltungen sind beispielsweise von Telefonen oder Babyphonen zur Modulation von Tonfrequenzsignalen auf die Betriebsspannung bekannt.

Die Polarität oder Höhe der angelegten Spannung kann weitaus schneller als die thermische Trägheit des Systems umgeschaltet werden, sodass das Umschalten zwischen Heizspannung U_{H} und Messspannung U_{M} praktisch keine Temperaturänderung zur Folge hat.

Fig. 4 zeigt einen Schnitt durch die Ausführungsform eines Heizdrahtes 8. Ein Metalldraht 21 ist in eine Isolation 22 eingebettet. Die Isolierung hat einen sternförmigen Querschnitt mit fünf dreieckförmigen Strahlen und ist damit invariant gegenüber Drehungen um 72°. Auch der Metalldraht 21 kann einen sternförmigen Querschnitt aufweisen. Jeder Strahl bildet eine Erhebung die längs des Drahtes entlang läuft. Die Erhebungen können auch schraubenförmig um die Mantelfläche herumlaufen, wobei die Länge eines Umlaufs typischerweise ein mehrfaches des Umfangs der Isolierung beträgt. Zweck der sternförmigen Isolierung ist, die Oberfläche des Drahtes zu vergrößern und so den Wärmewiderstand zur umgebenden Luft zu verringern. Außerdem soll selbst bei abgeknicktem Schlauch der Heizdraht möglichst auf allen Seiten von Luft umströmt werden, um nicht zu überhitzen und in den umgebenden Schlauch einzuschmelzen. Die dreieckförmigen Strahlen des Querschnitts spreizen dabei die Knickstelle eines Schlauches wobei die Berührungsfläche zwischen Schlauch und Isolierung klein ist und damit der Wärmewiderstand groß bleibt. Der Metalldraht 21 kann einen Durchmesser von etwa 0,3 mm und ein Kreis, der die Spitzen des Querschnitts gerade umschließt, einen Durchmesser von 1 mm haben.

Fig. 6 zeigt einen Schnitt durch einen Schlauch, wobei es sich um einen Gabelschlauch 3 oder einen Zuleitungsschlauch 5 handeln kann. Beide Schlaucharten unterscheiden sich typischerweise hauptsächlich durch ihren Durchmesser. Die innere Mantelfläche des Schlauches weist Vorsprünge 32 auf, die dazu dienen, den Mantel des Schlauches auch an Knickstellen zu spreizen, um trotz Knick den Luftfluss nicht völlig abzuschnüren. Am äußeren Umfang des Schlauches und/oder im Schlauchmaterial selbst, insbesondere in Vorsprüngen 32 sind Stabilisierungsfäden 31 bzw. 33 an- bzw. eingebracht, um eine Längsdehnung des Schlauches zu reduzieren. Die Stabilisierungsfäden 31 und 33 können während des Herstellungsprozesses in das Schlauchmaterial, insbesondere in Vorsprünge 32 eingebracht werden. Die Stabilisierungsfäden 31 und 33 können aus künstlichem oder natürlichem Faserwerkstoff, Kunststoff oder Metall bestehen. Der Grund für die Stabilisierungsfäden liegt darin, dass wärmebeständiges PVC zu steif ist und deswegen beispielsweise TPE oder Silicon eingesetzt werden müssen. Letztere Materialien sind stark dehnbar, was in Längsrichtung unerwünscht sein kann, weil dann auftretende Zugkräfte vom Heizdraht aufgenommen werden müssen und diesen und seine Anschlüsse mechanisch beanspruchen. Da die Schläuche bei Maximaldrücken von wenigen 100 Millibar betrieben werden, erscheint eine Stabilisierung in radialer Richtung nicht erforderlich.

Wenn die Stabilisierungsfäden, insbesondere die in Vorsprüngen 32, aus einem elektrisch leitfähigem Material, insbesondere aus Metall, evt. umgeben von einer thermisch beständigen, nicht notwendigerweise biokompatiblen, elektrischen Isolation, bestehen, können diese zur Beheizung eingesetzt werden und Heizdraht 8 ersetzen. Auf diese Weise können Probleme mit nicht-biokompatiblen Isolationsmaterialien umgangen werden.

Schließlich können Gabelschlauch 3 und/oder Zuleitungsschlauch 5 von einer thermischen Isolation 34 umgeben sein. Diese sollte nicht zu dick sein, da insbesondere ein dünner Gabelschlauch Komfort und eine dicke Isolation eine Komforteinbuße bedeutet. Andererseits kann eine Isolation die Oberfläche der Schläuche weich und damit angenehmer machen. Aus technischer Sicht hat die Isolation den Vorteil, dass sie die Heizleistung reduziert. Diese muss auch im Fehlerfall bei Ausfall der Leistungsregelung und bei Anliegen der gesamten Versorgungsspannung unter 15W bleiben. Ein Absenken der Heizleistung macht deshalb den Einsatz weniger genau tolerierter und damit billigerer Heizdrähte oder längerer Schläuche möglich. Die im Augenblick angedachten Nasenbrillen benötigen tatsächlich fast 15W maximale Heizleistung.

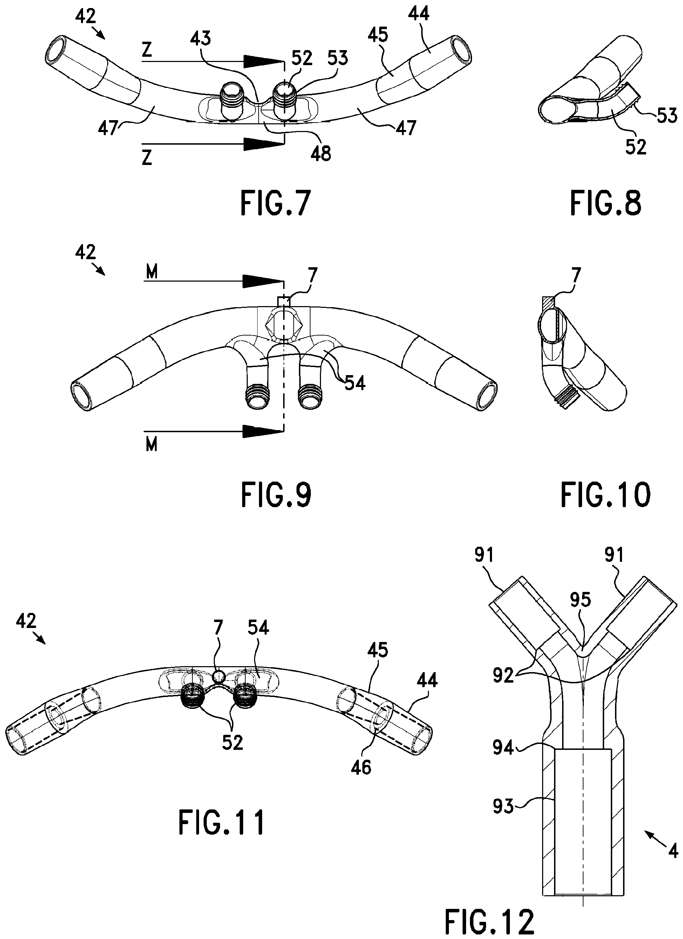

Die Figuren 7, 9 sowie 11 zeigen drei perspektivische Ansichten einer zweiten Ausführungsform eines Nasenstücks 42. Die Figuren 8 und 10 zeigen Schnitte entlang der Linien Z-Z bzw. M-M. Die zweite Ausführungsform des Nasenstücks 42 unterscheidet sich lediglich qualitativ von der Ausführungsform des Nasenstücks 2. Um die Geräuschemission zu reduzieren ist Nasenstück 2 bauchiger, d. h. die lichte Querschnittfläche nimmt von den Schlauchanschlüssen zu den Zinken hin stärker zu. Hierdurch wird die Strömungsgeschwindigkeit der Luft verringert, um die Geräuschemission gering zu halten. Die Verringerung des Strömungswiderstands durch die Vergrößerung der Querschnittfläche im Nasenstück ist vernachlässigbar, weil der Strömungswiderstand maßgeblich durch die Dicke von Gabelschlauch 3 bestimmt wird. Derzeit werden drei Prototypen mit einer jeweils unterschiedlichen Zunahme der Querschnittfläche vorbereitet. Messergebnisse liegen noch nicht vor.

Nasenstück 42 umfasst Schlauchanschlüsse 44, Schlauchübergangsbereiche 45, Verbindungsstücke 47, Zinken 52 mit ringförmigen Noppen 53 sowie einem zentralen Verbindungsstück 48. Wie man in Fig. 11 erkennt, befindet sich zwischen Schlauchübergangsbereichen 45 und Schlauchanschlüssen 44 je eine Innenradiusstufe 46, die gerade den Unterschied zwischen Innen- und Außenradius von Gabelschlauch 3 kompensiert, um einen möglichst glatten Übergang zwischen der Innenfläche von Gabelschlauch 3 und Nasenstück 42 zu erzielen. Zu diesem Zweck können an den Enden von Gabelschlauch 3 die Vorsprünge 32 entfernt sein oder entsprechende Vorsprünge an der Innenfläche von Nasenstück 42 angeformt sein.

Wie ebenfalls in Fig. 11 zu erkennen ist, weitet sich die lichte Querschnittfläche im Schlauchübergangsbereich 45.

Wie in Fig. 9 gut zu erkennen ist, sind die Übergangsbereiche 54 zwischen Zinken 52 und Verbindungsstücken 47 großzügig ausgerundet, um Geräuschemission zu reduzieren. Bei dem Prototypen beträgt beispielsweise dieser Radius außen 4,3 mm. Der Außendurchmesser der Zinken beträgt in der Nähe des Verbindungsstücks 5,5 mm und in der Nähe der Öffnung 5 mm. Die Wandstärke beträgt etwa 0,5 mm.

Der Übergangsbereich zwischen dem zentralen Verbindungsstück 48 und den Zinken 52 ist ebenfalls abgerundet, wobei der Außenradius ebenfalls im Bereich zwischen 4 und 5 mm liegt.

Die Einbuchtung 43 im zentralen Verbindungsstück 48 ist in den Fig. 8 und 10 im Schnitt und in Fig. 9 in einer Draufsicht dargestellt. Sie dient dazu, zwischen der linken und rechten Seite der Nasebrille einen definierten Strömungswiderstand einzustellen. Wie in Figur 1 gezeigt, ist die Luftbrille spiegelsymmetrisch. Dies trifft auch in den meisten Fällen für den Benutzer zu. Solange Spiegelsymmetrie gegeben ist, strömt durch das zentrale Verbindungsstück 48 keine Luft. Die Symmetrie kann beispielsweise dadurch gebrochen werden, dass entweder der linke oder rechte Gabelschlauch 3 abgeknickt ist oder der Benutzer Schnupfen hat und deshalb ein Nasenloch zugeschwollen ist. Im ersteren Fall ist es einerseits wünschenswert, dass beide Zinken über den noch offenen Schlauch versorgt werden. Andererseits ist ja der abgeknickte Gabelschlauch nicht vollständig verschlossen. Je höher der Druckabfall am abgeknickten Gabelschlauch, desto größer der Kühlluftfluss für Heizdraht 8. Um den Druckabfall am abgeknickten Gabelschlauch leicht zu erhöhen, kann ein Druckabfall am zentralen Verbindungsstück 48 wünschenswert sein. Falls ein Nasenloch zugeschwollen ist, ist es wünschenswert, mehr Luft über den anderen Zinken zu applizieren. Auch in diesem Fall ist ein Luftfluss durch das zentrale Verbindungsstück 48 wünschenswert.

In den Figuren 9, 10 sowie 11 ist auch der Temperatursensor 7 dargestellt.

In Fig. 12 ist das Y-Stück 4 vergrößert dargestellt. Man erkennt oben die beiden Gabelschlauchanschlüsse 91 und unten den Zuleitungsschlauchanschluss 93. Der Übergangsbereich 95 zwischen den beiden Gabelschlauchanschlüssen ist abgerundet und weist bei einer Ausführungsform einen Radius von 1 mm auf. Zum Vergleich weisen bei dieser Ausführungsform die Gabelschläuche und der Zuleitungsschlauch einen Innenradius (ohne Vorsprünge 32) von 3 bzw. 5 mm auf. Die Abrundung von Übergangsbereich 95 ist insbesondere dann wichtig, wenn beispielsweise wegen Abknicken eines Gabelschlauches unsymmetrische Strömungsverhältnisse vorliegen. Alle Anschlüsse weisen Innenradiusstufen 92 und 94 auf, um den Unterschied zwischen Innenradius und Außenradius der angeschlossenen Schläuche zu kompensieren. Die Innenradiusstufen können entweder den Vorsprüngen 32 in den angeschlossenen Schläuchen entsprechende Vorsprünge aufweisen und/oder die Vorsprünge 32 können an den Schlauchenden entfernt sein.

Obwohl die Erfindung oben im Zusammenhang mit dem Gas Luft erläutert wurde, kann natürlich auch jedes andere, atembare Gasgemisch verwendet werden. Ganz abgesehen davon ist die Zusammensetzung von Luft beispielsweise hinsichtlich ihres Wasser- und Sauerstoffanteils nicht exakt festgelegt.

Den oben beschriebenen Ausführungsformen kann folgende Merkmalsanalyse zu Grunde gelegt werden:
Eine 1. Luftbrille für ein Antischnarch- oder PAP-Gerät mit:
   einem Gabelschlauch (3), der mit Öffnungen (12; 52) pneumatisch verbunden ist, die so ausgestaltet und positioniert sind, dass über diese Öffnungen (12; 52) Luft in die Nase eines Benutzers appliziert werden kann,
   gekennzeichnet durch:
      einen Heizdraht (8, 31, 33), der im Gabelschlauch (3) verläuft, so dass der Heizdraht (8) die durch den Gabelschlauch (3) zugeführte Luft erwärmen kann.
Eine 2. Luftbrille gemäß der 1. Luftbrille, dadurch gekennzeichnet, dass ein Temperatursensor (7) in der Nähe der Öffnungen (12; 52) angebracht ist, sodass der Temperatursensor (7) die Temperatur der über die Öffnungen (12; 52) applizierten Luft messen kann.
Eine 3. Luftbrille gemäß der 2. Luftbrille, dadurch gekennzeichnet, dass der Temperatursensor (7) mit dem Heizdraht (8) so verbunden ist, dass er über den Heizdraht (8) mit elektrischer Energie versorgt werden kann und das Temperatursignal des Temperatursensors (7) auch über den Heizdraht (8) ausgelesen werden kann.
Eine 4. Luftbrille gemäß der 3. Luftbrille, dadurch gekennzeichnet, dass der Temperatursensor (7) ein digitaler Temperatursensor ist, der sein Temperatursignal auf die Spannung aufmoduliert, die dem Temperatursensor (7) über den Heizdraht (8) zugeführt wird.
Eine 5. Luftbrille gemäß einer der obigen Luftbrillen, dadurch gekennzeichnet, dass der Heizdraht (8) einen drahtförmigen Metallkern (21) aufweist, der von einer Isolierung (22) umgeben ist, deren äußere Mantelfläche Erhöhungen und Vertiefungen aufweist.
Eine 6. Luftbrille gemäß der 5. Luftbrille, dadurch gekennzeichnet, dass die Mantelfläche der Isolierung (22) etwa in Längsrichtung des Heizdrahtes (8) verlaufende Erhöhungen mit dreieckförmigen Querschnitten aufweist, sodass die Isolierung (22) insgesamt einen sternförmigen Querschnitt aufweist.
Eine 7. Luftbrille gemäß der 5. oder 6. Luftbrille, dadurch gekennzeichnet, dass auch der Metallkern (21) auf seiner Mantelfläche Erhöhungen und Vertiefungen aufweist.
Eine 8. Luftbrille gemäß einer der obigen Luftbrillen, dadurch gekennzeichnet, dass der Gabelschlauch (3) Stabilisierungsfäden (31, 33) aufweist.
Eine 9. Luftbrille gemäß einer der obigen Luftbrillen, dadurch gekennzeichnet, dass zwei Stücke des Gabelschlauches (3) an einem steckerseitigen Ende mechanisch zu einem Doppellumenschlauch (13) verbunden sind.
Eine 10. Luftbrille gemäß einer der obigen Luftbrillen, dadurch gekennzeichnet, dass der Gabelschlauch (3) pneumatisch mit einem pneumatischen Steckerteil (10) eines Steckers (6) und der Heizdraht (8) elektrisch mit einem elektrischen Steckerteil (9) des Steckers (6) verbunden sind.
Eine 11. Luftbrille gemäß einer der obigen Luftbrillen, dadurch gekennzeichnet, dass die Öffnungen durch Zinken (12, 52) etwa in der Mitte eines Nasenstücks (2, 42) gebildet werden, wobei ein linkes Stück des Gabelschlauches (3) an der linken Seite des Nasenstücks (2, 42) und ein rechtes Stück des Gabelschlauches (3) an der rechten Seite des Nasenstücks (2, 42) pneumatisch angeschlossen sind und der Heizdraht (8, 31, 33) vom linken Stück des Gabelschlauches (3) durch das Innere des Nasenstücks (2, 42) zum rechten Stück des Gabelschlauches (3) verläuft.
Ein 1. Nasenstück für eine Luftbrille insbesondere gemäß einer der obigen Luftbrillen, mit:
   einer Verbindungsstelle (44) zum Anbringen eines Gabelschlauches (3);
   dadurch gekennzeichnet, dass die Verbindungsstelle eine Innenradiusstufe (46) an einem Ende der Verbindungsstelle (44) aufweist, deren Höhe gerade der halben Differenz zwischen Innen- und Außendurchmesser des Gabelschlauches (3) entspricht, sodass sich bei Anbringen eines Gabelschlauches (3) an der Verbindungsstelle (44) ein glatter Übergang zwischen dem Inneren des Gabelschlauches (3) und dem Inneren des Nasenstücks ergibt.
Ein 2. Nasenstück, insbesondere gemäß dem 1. Nasenstück, für eine Luftbrille insbesondere gemäß einer der 1. bis 11. Luftbrillen, mit:
   einem Zinken (12; 52) zum Applizieren von Luft in ein Nasenloch eines Benutzers;
   einer Verbindungsstelle (44) zum Anbringen eines Gabelschlauches (3); und
   einem Verbindungsstück (47), das den Zinken (12; 52) mit der Verbindungsstelle (44) mechanisch und pneumatisch verbindet,
   dadurch gekennzeichnet, dass der Übergangsbereich (54) zwischen dem Zinken (12; 52) und dem Verbindungsstück (47) einen Radius in einer durch den Zinken und das Verbindungsstück (47) festgelegten Ebene aufweist, der größer als der Radius des Zinken (12; 52) ist.
Ein 3. Nasenstück, insbesondere gemäß dem 1. oder 2. Nasenstück, für eine Luftbrille insbesondere gemäß einer der 1. bis 11. Luftbrillen, mit:
   zwei Zinken (12; 52) zum Applizieren von Luft in je ein Nasenloch eines Benutzers;
   einem zentralen Verbindungsstück (48), das die beiden Zinken (12; 52) mechanisch und pneumatisch verbindet;
   zwei Schlauchanschlüsse (44); und
   zwei Verbindungsstücke (47), wobei jedes Verbindungsstück je einen Zinken (12; 52) mit einem Schlauchanschluss (44) mechanisch und pneumatisch verbindet,
   dadurch gekennzeichnet, dass
   das zentrale Verbindungsstück (48) eine Einbuchtung (43) aufweist, so dass die Fläche des lichten Querschnitts des zentralen Verbindungsstücks (48) kleiner als die Fläche der lichten Querschnitte der beiden Verbindungsstücke (47) ist.
Ein 4. Nasenstück, insbesondere gemäß einem der 1. bis 3. Nasenstücke, für eine Luftbrille insbesondere gemäß einer der 1. bis 11. Luftbrillen, mit:
   zwei Zinken (12; 52) zum Applizieren von Luft in je ein Nasenloch eines Benutzers;
   einem zentralen Verbindungsstück (48), das die beiden Zinken (12; 52) mechanisch und pneumatisch verbindet;
   dadurch gekennzeichnet, dass
   der Übergangsbereich zwischen dem zentralen Verbindungsstück (48) in einer durch die beiden Zinken (12; 52) definierten Ebene abgerundet ist, wobei der Radius dieses Übergangsbereichs größer als der Radius der Zinken ist.
Ein 1. Y-Stück für eine Luftbrille insbesondere gemäß einer der 1. bis 8. Luftbrillen, mit:
   zwei Gabelschlauchanschlüssen (91); und
   einem Zuleitungsschlauchanschluss (93), wobei das Y-Stück alle drei Schlauchanschlüsse mechanisch und pneumatisch verbindet,
   dadurch gekennzeichnet, dass
   jeder der beiden Gabelschlauchanschlüsse (91) eine Innenradiusstufe (92) an einem Ende des Gabelschlauchanschlusses (91) aufweist, deren Höhe gerade der halben Differenz zwischen Innen- und Außendurchmesser des Gabelschlauches (3) entspricht, sodass sich bei Anbringen eines Gabelschlauches (3) an einem Gabelschlauchanschluss (91) ein glatter Übergang zwischen dem Inneren des Gabelschlauches (3) und dem Inneren des Y-Stücks (4) ergibt.
Ein 2. Y-Stück gemäß dem 1. Y-Stück, dadurch gekennzeichnet, dass der Zuleitungsschlauchanschluss (93) eine Innenradiusstufe (94) an einem Ende des Zuleitungsschlauchanschlusses (93) aufweist, deren Höhe gerade der halben Differenz zwischen Innen- und Außendurchmesser des Zuleitungsschlauches (5) entspricht, so dass sich bei Anbringen eines Zuleitungsschlauches (5) am Zuleitungsschlauchanschluss (93) ein glatter Übergang zwischen dem Inneren des Zuleitungsschlauches (5) und dem Inneren des Y-Stücks (4) ergibt.
Ein 3. Y-Stück gemäß dem 1. oder 2. Y-Stück, dadurch gekennzeichnet, dass der Übergangsbereich (95) zwischen den beiden Gabelschlauchanschlüssen (91) im Inneren des Y-Stücks (4) abgerundet ist, wobei der Radius in diesem Übergangsbereich (95) in einer durch die beiden Gabelschlauchanschlüsse (91) definierten Ebene größer als ein Zehntel des lichten Querschnitts eines Gabelschlauchanschlusses (91) ist.
Ein 1. Verfahren zur Vermeidung von Kondensation in einer Luftbrille mit:
   Zuführen (6) eines Gases zur Luftbrille; und
   Applizieren des Gases durch Öffnungen (12; 52) in der Luftbrille;
   gekennzeichnet durch:
      Heizen (8) des Gases während es durch die Schläuche der Luftbrille strömt.
Ein 2. Verfahren gemäß dem 1. Verfahren, gekennzeichnet durch:
   Messen (7) der Temperatur in der Nähe der Öffnungen (12; 52) zum Applizieren des Gases; und
   Steuern der Heizleistung, so dass eine Kondensation in der Luftbrille vermieden wird.
Ein 3. Verfahren gemäß dem 1. oder 2. Verfahren, dadurch gekennzeichnet, dass ein Temperatursensor (7), der die Temperatur in der Nähe der Öffnungen (12; 52) misst, über Heizdrähte (8) zum Heizen des Gases mit elektrische Energie versorgt wird und die Heizdrähte (8) zur Übertragung des Sensorsignals verwendet werden.

### Bezugszeichenliste

- 1: Luftbrille
- 2: Nasenstück
- 3: Gabelschlauch
- 4: Y-Stück
- 5: Zuleitungsschlauch
- 6: Stecker
- 7: Temperatursensor
- 8: Heizdraht
- 9: elektrisches Steckerteil
- 10: pneumatisches Steckerteil
- 11: Klammer
- 12: Zinke
- 13: Doppellumenschlauch
- 14: Schelle
- 16: Innenradiusstufe
- 17: zusätzliches Lumen
- 18: Trennwand
- 19: Temperaturschalter
- 21: Metalldraht
- 22: Isolation
- 31: Stabilisierungsfaden
- 32: Vorsprung
- 33: Stabilisierungsfaden
- 34: thermische Isolation
- 42: Nasenstück
- 43: Einbuchtung
- 44: Schlauchanschluss
- 45: Schlauchübergangsbereich
- 46: Innenradiusstufe
- 47: Verbindungsstück
- 48: zentrales Verbindungsstück
- 52: Zinke
- 53: Noppe
- 54: Zinkenübergangsbereich
- 91: Gabelschlauchanschluss
- 92: Innenradiusstufe
- 93: Zuleitungsschlauchanschluss
- 94: Innenradiusstufe
- 95: Übergangsbereich

## Patentansprüche

1. Nasenstück (42), für eine Luftbrille mit:
zwei Zinken (12, 52) zum Applizieren von Luft in je ein Nasenloch eines Benutzers;
zwei Verbindungsstellen (44) zum Anbringen je eines Endes eines Gabelschlauches (3); und einem Verbindungsstück, das die Zinken (12, 52) mit den zwei Verbindungsstellen (44) mechanisch verbindet,
wobei das Verbindungsstück umfasst:
ein zentrales Verbindungsstück (48), das die beiden Zinken (12, 52) mechanisch und pneumatisch verbindet; und zwei Verbindungsstücke (47), wobei jedes Verbindungsstück (47) je einen Zinken (12, 52) mit einer Verbindungsstelle (44) mechanisch und pneumatisch verbindet,
und wobei der Übergangsbereich (54) zwischen jedem der Zinken (12, 52) und dem Verbindungsstück (47) einen Radius in einer durch den Zinken und das Verbindungsstück (47) festgelegten Ebene aufweist, der größer als der Radius des Zinken (12, 52) ist,
**dadurch gekennzeichnet,**
**dass** das zentrale Verbindungsstück (48) eine Einbuchtung (43) aufweist, so dass die Fläche des lichten Querschnitts des zentralen Verbindungsstücks (48) kleiner als die Fläche der lichten Querschnitte der beiden Verbindungsstücke (47) ist.

2. Nasenstück (42) gemäß Anspruch 1,
**dadurch gekennzeichnet,**
**dass** das Nasenstück (42) zwei Schlauchanschlüsse (44) umfasst, wobei jeder Schlauchanschluss (44) eine Innenradiusstufe (46) an einem Ende des Schlauchanschlusses (44) aufweist, deren Höhe gerade der halben Differenz zwischen Innen und Außendurchmesser des entsprechenden Endes des Gabelschlauches (3) entspricht, sodass sich bei Anbringen eines Endes des Gabelschlauches (3) an dem Schlauchanschluss (44) ein glatter Übergang zwischen dem Inneren des Gabelschlauches (3) und dem Inneren des Nasenstücks ergibt.

3. Nasenstück (42) gemäß Anspruch 2,
**dadurch gekennzeichnet,**
**dass** ein Übergangsbereich zwischen dem zentralen Verbindungsstück (48) in einer durch die beiden Zinken (12, 52) definierten Ebene abgerundet ist, wobei der Radius dieses Übergangsbereichs größer als der Radius der Zinken ist.

## Claims

1. A nosepiece (42) for a nasal cannula, comprising:
two prongs (12, 52) for administering air into each nostril of a user;
two connections points (44) for mounting each of the ends of a forked tube (3); and a connection piece which mechanically connects the prongs (12, 52) to the two connection points (44),
the connection piece comprising:
a central connection piece (48) mechanically and pneumatically connecting the two prongs (12, 52); and two connection pieces (47), each connection piece (47) mechanically and pneumatically connecting one prong (12, 52) to one connection point (44),
and wherein the transition region (54) between each of the prongs (12, 52) and the connection piece (47) has a radius in a plane which is defined by the prong and the connection piece (47), the radius being larger than the radius of the prong (12, 52),
**characterized in that**
the central connection piece (48) includes an indentation (43) so that the area of the clear cross-section of the central connection piece (48) is smaller than the area of the clear cross-sections of the two connection pieces (47).

2. The nosepiece (42) according to claim 1,
**characterized in that**
the nosepiece (42) comprises two tube connections (44), each tube connection (44) including an internal radius step (46) at one end of the tube connection (44), the height of which just about corresponds to half the difference between the inner and outer diameter of the corresponding end of the forked tube (3) so as to obtain an even transition between the interior of the forked tube (3) and the interior of the nosepiece upon mounting one end of the forked tube (3) at the tube connection (44).

3. The nosepiece (42) according to claim 2,
**characterized in that**
a transition region between the central connection piece (48) is rounded off in a plane which is defined by the two prongs (12, 52), wherein the radius of said transition region is larger than the radius of the prongs.

## Revendications

1. Partie de nez (42), pour une canule nasale ayant :
deux pointes (12, 52) pour appliquer de l'air dans chacune des narines d'un utilisateur ;
deux points de connexion (44) pour attacher chacune des extrémités d'un flexible fourchu (3) ; et
une pièce de connexion qui relie les pointes (12, 52) aux deux points de connexion (44) de façon mécanique,
la pièce de connexion comprenant :
une pièce de connexion centrale (48) qui relie les deux pointes (12, 52) de façon mécanique et pneumatique ; et
deux pièces de connexion (47), chaque pièce de connexion (47) reliant une pointe (12, 52) à un point de connexion (44) de façon mécanique ou pneumatique,
et la zone de transition (54) comprenant un radius entre chacune des pointes (12, 52) et la pièce de connexion (47) dans un plan défini par la pointe et la pièce de connexion (47), ledit radius étant plus grand que le radius de la pointe (12, 52),
**caractérisée en ce que**
la pièce de connexion centrale (48) comprend une encoche (43), de sorte que la surface de la section transversale libre de la pièce de connexion centrale (48) soit plus petite que la surface des sections transversales libres des deux pièces de connexion (47).

2. Partie de nez (42) selon la revendication 1,
**caractérisée en ce que**
la partie de nez (42) comprend deux raccords de flexible, chaque raccord de flexible (44) comprenant une saillie de radius intérieur (46) à une extrémité du raccord de flexible (44), dont l'hauteur à peine correspond à la moitié de la différence entre le diamètre extérieur et le diamètre intérieur de l'extrémité correspondante du flexible fourchu (3), de sorte qu'une transition plane se produise entre l'intérieur du flexible fourchu (3) et l'intérieur de la pièce de nez quand une extrémité du flexible fourchu (3) est attachée au raccord de flexible (44).

3. Partie de nez (42) selon la revendication 2,
**caractérisée en ce qu'**
une zone de transition entre la pièce de connexion centrale (48) dans un plan défini par les deux pointes (12, 52) est arrondie, le radius de cette zone de transition étant plus grand que le radius des pointes.
